Europäisches Patentamt

European Patent Office

Office européen des brevets

(11) Veröffentlichungsnummer: **0 332 718**

**A1**

# EUROPÄISCHE PATENTANMELDUNG

(21) Anmeldenummer: 88104006.7

(22) Anmeldetag: 14.03.88

(51) Int. Cl.⁴: **A61F 15/00 , A45D 44/00**

(43) Veröffentlichungstag der Anmeldung:
20.09.89 Patentblatt 89/38

(84) Benannte Vertragsstaaten:
AT BE CH DE ES FR GB GR IT LI LU NL SE

(71) Anmelder: **Roescheisen GmbH & Co.**
**Scheffeltgasse 3**
**D-7900 Ulm(DE)**

(72) Erfinder: **Kreuzer Heinz**
**c/o Müller GmbH & Co KG Steeger Strasse**
**32**
**5250 Engelskirchen 1(DE)**

(74) Vertreter: **Patentanwälte Grünecker,**
**Kinkeldey, Stockmair & Partner**
**Maximilianstrasse 58**
**D-8000 München 22(DE)**

(54) **Vorrichtung zum Spenden von Wattekugeln.**

(57) Die Erfindung betrifft eine Vorrichtung zum Spenden von Wattekugeln (17) mit einem Einsatz (2) zur Aufnahme der Wattekugeln und einem Gehäuse (1) zur Aufnahme des Einsatzes, wobei das Gehäuse eine Gehäusewandung, einen Gehäuseboden, eine Entnahmeöffnung (16) für die Wattekugeln sowie eine Einsetzöffnung (20) aufweist, durch die der Einsatz in das Gehäuse einsetzbar ist, und der Einsatz ein Einsatzgehäuse mit einer Durchtrittsöffnung (30) für die Wattekugeln umfaßt. Um eine einfachere Konstruktion und leichtere Handhabung, insbesondere beim Auswechseln des Einsatzes, zu erzielen, ist in einer Vorderwand der Gehäusewandung (4) die Einsetzöffnung sowie in wenigstens einer der sich die Vorwand anschließenden Gehäuseflanken (5) eine Flankenöffnung (21) ausgebildet.

FIG.2

## Vorrichtung zum Spenden von Wattekugeln

Die Erfindung betrifft eine Vorrichtung zum Spenden von Wattekugeln mit einem Einsatz zur Aufnahme der Wattekugeln und einem Gehäuse zur Aufnahme des Einsatzes, wobei das Gehäuse eine Gehäusewandung, einen Gehäuseboden, eine Entnahmeöffnung für die Wattekugeln sowie eine Einsetzöffnung aufweist, durch die der Einsatz in das Gehäuse einsetzbar ist, und der Einsatz ein Einsatzgehäuse mit einer Durchtrittsöffnung für de Wattekugeln umfaßt.

Aus der US-PS 21 36 352 ist eine derartige Vorrichtung bekannt, deren Gehäuse eine zylindrischen Gehäusewandung umfaßt, an deren oberen Ende die Entnahmeöffnung und an deren unteren Ende die Einsetzöffnung angeordnet sind. Der Gehäuseboden ist auf ein am unteren Ende der Gehäusewandung ausgebildetes Gewinde aufschraubbar, um die Einsetzöffnung zu verschließen.

Diese bekannte Vorrichtung weist mehrere Nachteile auf. Das Auswechseln des Einsatzes ist umständlich und zeitraubend, da jedes Mal der Gehäuseboden von der Gehäusewandung abgeschraubt und dann wieder aufgeschraubt werden muß. Wattekugeln, die zwischen den Einsatz und die Gehäusewandung gelangen, verhindern oftmals das Entfernen des Einsatzes unter dem Einfluß der Schwerkraft und erfordern die Verwendung von Hilfsmitteln, um den Einsatz aus dem Gehäuse herauszudrücken. Ferner besteht die Gefahr, daß die an der Gehäusewandung und am Gehäuseboden ausgebildeten Gewindegänge beim Zusammenschrauben der beiden Vorrichtungsteile beschädigt werden. Weiterhin ist es leicht möglich, daß der abgeschraubte Gehäuseboden verloren geht. Außerdem ist die Konstruktion aufgrund ihrer mehrteiligen und mit Gewindegängen versehenen Konstruktion sehr kostenaufwendig.

Es ist Aufgabe der Erfindung, eine Vorrichtung der eingangs genannten Art zu schaffen, die sich durch einfachere Konstruktion und leichtere Handhabung, insbesondere beim Auswechseln des Einsatzes, auszeichnet.

Dies wird erfindungsgemäß dadurch erreicht, daß in einer Vorderwand der Gehäusewandung die Einsetzöffnung sowie in wenigstens einer der sich an die Vorderwand anschließenden Gehäuseflanken eine Flankenöffnung ausgebildet ist.

Bei der erfindungsgemäßen Vorrichtung wird der Einsatz sehr leicht und schnell in die stets offene Einsetzöffnung eingesetzt, so daß der Gehäuseboden nicht mehr abgeschraubt werden muß und dann verloren gehen kann. Es entfällt auch das Wiederaufschrauben des Gehäusebodens mit der Gefahr der Gewindebeschädigung. Das Entfernen des Einsatzes erfolgt in einfacher Weise dadurch, daß der Benutzer denselben durch die Flankenöffnung hindurch erfaßt und durch die Einsetzöffnung hindurch nach außerhalb des Gehäuses verschiebt. Dabei wird auch der Widerstand von Wattekugeln, die zwischen Einsatz- und Gehäusewandung gelangt sind, sehr leicht überwunden. Die Flankenöffnung weist insofern eine Doppelfunktion auf, als sie gleichzeitig eine Grifföffnung darstellt, welche verhindert, daß die Vorrichtung beim Anheben durch den Benutzer aus dessen Händen rutscht. Durch den Wegfall der Gewinde an der Gehäusewandung und dem Gehäuseboden und durch die Möglichkeit, das Gehäuse einteilig auszubilden, ergibt sich insgesamt eine einfacherere und damit kostengünstigere Konstruktion der Vorrichtung.

Besonders vorteilhaft es, wenn sich die Einsetzöffnung sowohl über die Gesamtbreite als auch die Gesamthöhe der Vorderwand erstreckt, d.h. die Gesamtfläche der Vorderwand einnimmt und diese somit ersetzt. Dabei gestaltet sich das Auswechseln des Einsatzes besonders einfach. Beispielsweise ist es auch möglich, die in der Einsetzöffnung sichtbare vordere Einsatzwand zumindest teilweise durchsichtig zu gestalten, um den Füllgrad des Einsatzes jederzeit kontrollieren zu können.

Zur weiteren Verbesserung des Entfernens des Einsatzes ist es günstig, die Flankenöffnung als eine in die Einsetzöffnung mündende Aussparung auszubilden. Dabei kann in jeder Gehäuseflanke eine Flankenöffnung ausgebildet sein, die gemäß einer Weiterbildung der Erfindung einander gegenüberliegend angeordnet sind. Damit läßt sich der Einsatz aus dem Gehäuse in optimaler Weise entfernen, da der Einsatz von beiden Seiten durch die Aussparungen hindurch entsprechend fest gehalten und in einer einzigen glatten Bewegung gegen jeden Widerstand aufgrund eingeklemmter Wattekugeln (zwischen Gehäusewandung und Einsatz) durch die Einsetzöffnung herausgezogen werden kann. Infolge der zusätzlichen Funktion der Flankenöffnungen als Grifföffnungen ergibt sich bei gegenüberliegender Ausbildung in beiden Gehäuseflanken auch eine optimale Grifffestigkeit.

Die Flankenöffnungen bringen auch den Vorteil, daß man sie benutzen kann, um das Gehäuse zum Entnehmen von Wattekugeln zu neigen, während es auf einer Fläche steht. Es genügt mit der Hand in die Flankenöffnungen zu greifen, um das Gehäuse einseitig hochzuheben.

Vorzugsweise ist das Gehäuse mit einem Zusatzgewicht versehen, um dessen Standfestigkeit zu erhöhen. Dieses Zusatzgewicht kann an einer der Einsetzöffnung gegenüberliegenden Gehäuserückwand angebracht sein. Dabei ist es vorteilhaft, die Gehäuserückwand mit einer äußeren und einer inneren Rückwand doppelwandig auszubilden und das Zusatzgewicht im Raum zwischen beiden Rückwänden anzuordnen.

Um das Verschieben der Wattekugeln in Richtung der Durchtrittsöffnung und der Entnahmeöffnung und auf diese Weise die leichte Entnahme der Wattekugeln zu gewährleisten, kann im Einsatz ein in Richtung zur Durchtrittsöffnung verschiebbares Bodenelement angeordnet sein. Die Verschiebung dieses Bodenelementes kann beispielsweise durch eine Gehäuseboden abgestüzte Feder erfolgen. Um eine manuelle Verschiebung des Bodenelementes mit dem Vorteil individuell anpaßbarer Durckausübung zu erzielen, kann gemäß einer Weiterbildung der Erfindung im Einsatzboden und im Gehäuseboden je eine Bodenöffnung als Durchgreiföffnungen zur Druckausübung auf das Bodenelement ausgebildet sein. Vorteilhafterweise sind diese Bodenöffnungen bei in das Gehäuse eingesetztem Einsatz aufeinander ausgerichtet.

Nachstehend ist die Erfindung anhand eines Ausführungsbeispiels unter Bezugnahme auf die Zeichnung näher beschrieben. Es zeigen:

Fig.1 eine perspektivische Darstellung eines Ausführungsbeispiels der erfindungsgemäßen Vorrichtung mit in das Gehäuse eingesetztem Einsatz, jedoch der besseren Darstellung halber ohne Dekkel und ohne Wattekugeln,

Fig.2 eine perspektivische Darstellung der in Fig. 1 gezeigten Vorrichtung mit zum Teil in das Gehäuse eingeschobenem Einsatz, gefüllt mit Wattekugeln,

Fig.3 eine perspektivische Darstellung der in Fig. 1 gezeigten Vorrichtung ohne Einsatz,

Fig.4 eine Seitenansicht der in Fig. 1 gezeigten, jedoch durch einen Deckel verschlossenen Vorrichtung in Schnittdarstellung, und

Fig.5 die in Fig. 4 gezeigte Vorrichtung in gleicher Darstellung, lediglich mit geöffnetem Dekkel und nach oben verschobenem Bodenelement.

Die erfindungsgemäße Vorrichtung zum Spenden von Wattekugeln im hier gezeigten Ausführungsbeispiel besteht aus einem Gehäuse 1, einem Einsatz 2 und einem Deckel 3.

Das Gehäuse 1 umfaßt eine nahezu würfelförmige Gehäusewandung 4 mit zwei Gehäuseflanken 5, die durch einen Gehäuseboden 6, eine Gehäuserückwand 7 und eine Gehäusedeckwand 8 miteinander verbunden sind.

Die Gehäuserückwand 7 ist mit einer äußeren Rückwand 9 und einer im Abstand dazu angeordneten inneren Rückwand 10 doppelwandig ausgebildet. Im Zwischenraum 11 zwischen beiden Rückwänden 9 und 10 ist ein Zusatzgewicht 12 angeordnet, welches der Vorrichtung zum Spenden von Wattekugeln eine zusätzliche Standfestigkeit verleiht. Der verbleibende Raum 13 des Gehäuses 1 stellt den Aufnahmeraum für den Einsatz 2 dar. In einem mittleren Bereich der inneren Rückwand 10 ist ein Gummmipuffer 14 als Anschlag für den Einsatz 2 befestigt.

In dem den Aufnahmeraum 13 begrenzenden Teil des Gehäusebodens 6 ist eine kreisrunde Bodenöffnung 15 und im entsprechenden Teil der Gehäusedeckwand 8 eine rechteckige Entnahmeöffnung 16 für die Wattekugeln 17 ausgebildet. Beide Öffnungen 15, 16 sind aufeinander ausgerichtet.

Die Entnahmeöffnung 16 ist durch Stege 18, die mit gegenseitigem Abstand parallel zueinander angeordnet sind, teilverschlossen. Die Stege 18 sind um ihre jeweilige Längsachse drehbar in den Gehäuseflanken 5 direkt unterhalb dem die Entnahmeöffnung 16 begrenzenden Teil der Gehäusedeckwand 8 gelagert.

Das Gehäuse 1 weist keine der Gehäuserückwand 7 gegenüberliegende Vorderwand auf, so daß anstelle derselben eine lediglich von den Vorderkanten 19 des Gehäuses 1 begrenzte Einsetzöffnung 20 vorhanden ist. Die Einsetzöffnung 20 dient zum Einschieben des Einsatzes 2 in den Aufnahmeraum 13 und zum Entfernen aus demselben.

In den den Aufnahmeraum 13 begrenzenden Teilen der beiden Gehäuseflanken 5 ist je eine langgestreckte, in die Einsetzöffnung 20 einmündende Flankenöffnung 21 als Aussparung ausgebildet, die durch untere und obere Längskanten 22, 23 und eine diese Längskanten abgerundet verbindende Vertikalkante 25 begrenzt ist. Beide Aussparungen 21 verlaufen parallel zum und im gleichen Abstand vom Gehäuseboden 6 über etwa zwei Drittel der Länge der den Aufnahmeraum 13 begrenzenden Teilen der Gehäuseflanken 5.

Der Einsatz 2 umfaßt ein oben offenes Einsatzgehäuse bestehend aus zwei Einsatz-Seitenwänden 25, die durch einen Einsatzboden 26, eine Einsatzrückwand 27 und eine vordere Einsatzwand 28 miteinander verbunden sind. Die oberen Kanten 29 der Einsatz-Seitenwände 25, der Einsatzrückwand 27 und der vorderen Einsatzwand 28 begrenzen eine Durchtrittöffnung 30 für die Wattekugeln 17 im Einsatz 2. Die Durchtrittsöffnung 30 des Einsatzes 2 ist im wesentlichen von gleicher Form und Größe wie die Entnahmeöffnung 16 des Gehäuses 1.

Im Einsatzboden 26 ist eine kreisrunde Bodenöffnung 31 von etwa gleicher Größe wie die Bodenöffnung 15 im Gehäuseboden 6 augebildet.

Der Aufnahmeraum 13 im Gehäuse 1 und der Einsatz 2 sind in ihren Abmessungen derart aufeinander abgestimmt, daß der mit seiner Einsatzrückwand 27 bis zur Anlage an den Gummipuffer 14 in den Aufnahmeraum 13 eingeschobene Einsatz 2 mit seiner vorderen Einsatzwand 28 im wesentlichen bündig mit den Vorderkanten 19 des Gehäuses 1 abschließt. Dann sind auch die Bodenöffnungen 15 und 31 im Gehäuseboden 6 und im Einsatzboden 26 sowie die Durchtrittsöffnung 30 des Einsatzes 2 und die Entnahmeöffnung 16 des Gehäuses 1 sämtlich aufeinander ausgerichtet.

Der Einsatz 2 wird beim Verschieben innerhalb des Gehäuses 1 durch die Gehäuseflanken 5, den Gehäuseboden 6 und die Stege 18 geführt. Letztere setzen dieser Bewegung des Einsatzes 2 praktisch keinen Widerstand entgegen, das sie sich aufgrund ihrer Anordnung quer zur Bewegungsrichtung des Einsatzes 2 bei dessen Bewegung um ihre jeweilige Längsachse drehen.

Im Einsatz 2 ist ein Bodenelement 32 angeordnet, welches im wesentlichen die gleiche Größe und Form wie der Einsatzboden 24 des Einsatzes 2 aufweist, jedoch innerhalb des letzteren in Richtung zur Durchtrittsöffnung 30 verschiebbar ist.

In den beiden oberen Eckbereichen der äußeren Rückwand 9 des Gehäuses 1 ist je eine Öse 33 zur schwenkbaren Lagerung des Deckels 3 befestigt. Der Deckel 3 steht sowohl über die Vorderkanten 19 als auch über die äußere Rückwand 9 des Gehäuses 1 über.

Der Einsatz 2 ist als eine mit den Wattekugeln 17 gefüllte und mittels einer nicht dargestellten, über der Durchtrittsöffnung 30 angeordneten Folie steril verpackte Nachfüllpackung ausgebildet.

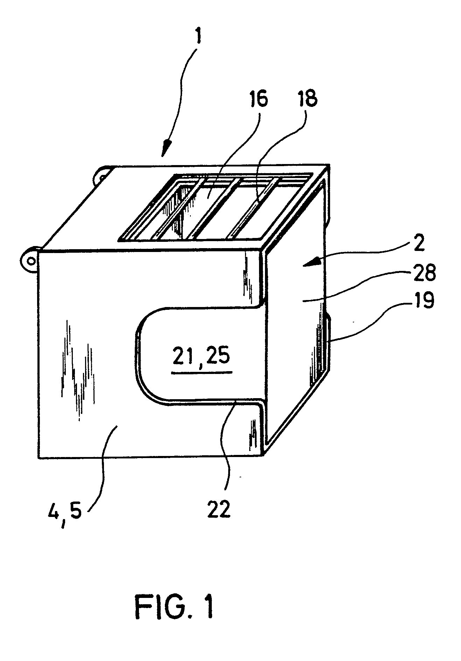

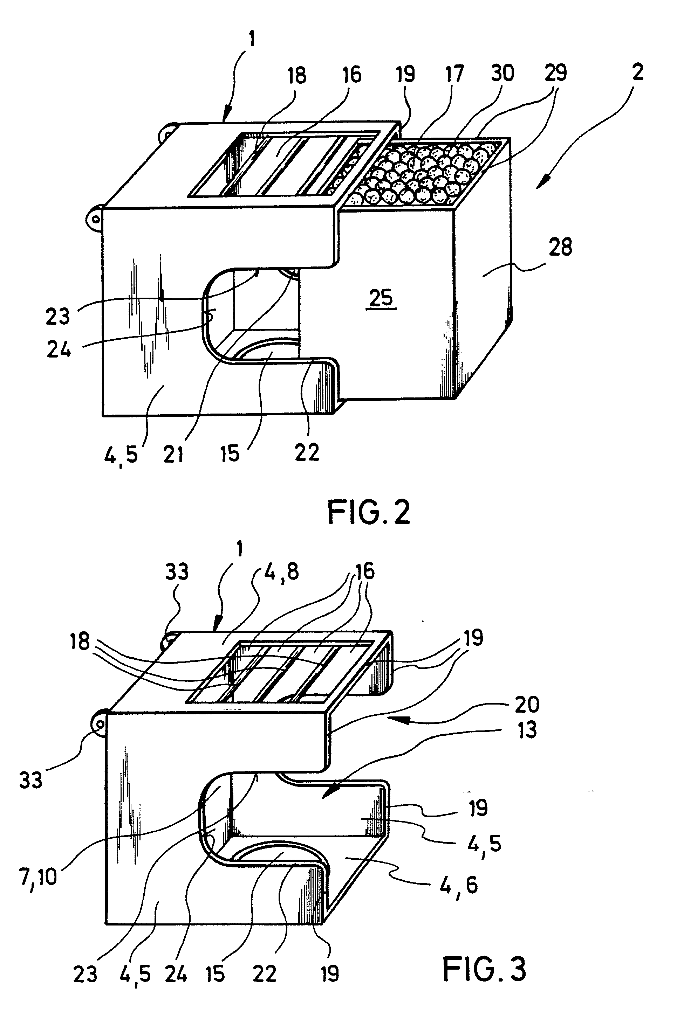

Um das in Fig. 3 dargestellte leere Gehäuse 1 mit dem Einsatz 2 zu füllen und damit betriebsbereit zu machen, ist es lediglich erforderlich, durch Entfernen der Folie die Durchtrittsöffnung 30 freizulegen und den Einsatz 2 in die Einsetzöffnung 20 des Gehäuses 1, wie in Fig. 2 gezeigt, einzusetzen und sodann in den Aufnahmeraum 13. bis zur Anlage an den Gummipuffer 14 einzuschieben. Damit befindet sich die Vorrichtung zum Spenden von Wattekugeln in ihrer in Fig. 1 gezeigten Betriebsstellung.

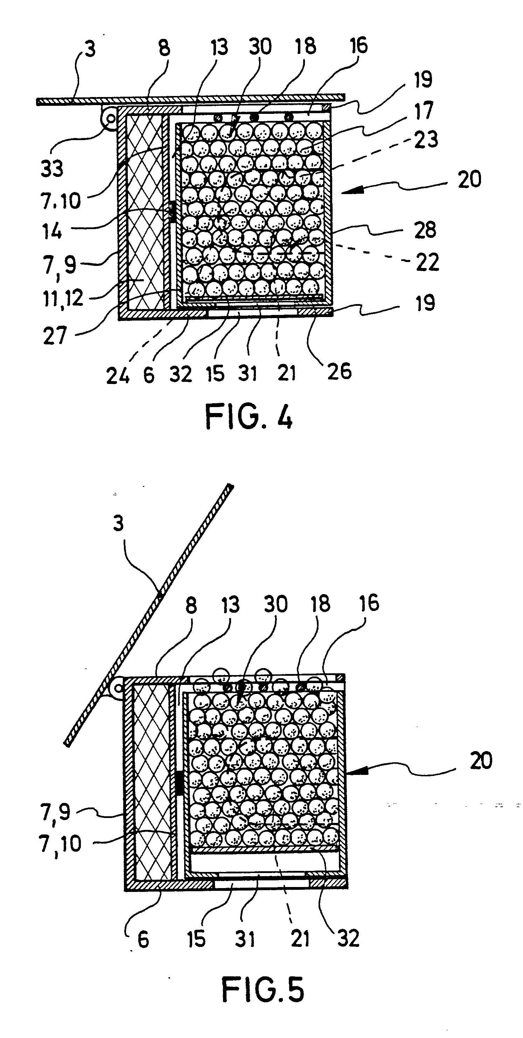

Der Benutzer kann nun durch einfache Druckausübung, beispielsweise mittels eines Fingers, durch die aufeinander ausgerichteten Bodenöffnungen 15 und 31 im Gehäuseboden 6 bzw. im Einsatzboden 26 das Bodenelement 32 so weit nach oben verschieben, bis die ersten Wattekugeln durch die Durchtrittsöffnung 30 und die Entnahmeöffnung 16 hindurchtreten, wie dies in Fig. 5 gezeigt ist. Dabei sind die Abstände zwischen den Stegen 18 sowie deren Durchmesser auf die Größe der Wattekugeln 18 so abgestimmt, daß letztere sich infolge des auf sie ausgeübten Druckes an den sich drehenden Stegen nach außen ohne Gefahr des Zerfaserns und des Verfilzens abwälzen können. Nach Schwenken des Deckels 3 in die in Fig. 5 gezeigte Stellung ist die Entnahmeöffnung 16 zugänglich und die Wattekugeln können beispielsweise mit Hilfe einer Pinzette entnommen werden. Anschließend kann der Deckel 3 in die in Fig. 4 gezeigte Stellung zurückverschwenkt werden, um die Entnahmeöffnung 16 zu verschließen.

Durch weiteres Verschieben des Bodenelementes 32 in Richtung zur Durchtrittsöffnung 30 werden die Wattekugeln 17 der Entnahmeöffnung 16 in dem Maße zugeführt, in welchem sie vom Benutzer entfernt werden. Sobald sich keine Wattekugeln 17 mehr im Einsatz 2 befinden, wird letzterer einfach dadurch aus dem Gehäuse 1 herausgezogen, daß der Benutzer durch die Aussparungen 21 hindurch beide Einsatz-Seitenwände 25 erfaßt und den Einsatz 2 sodann in einer einzigen glatten Bewegung aus dem Gehäuse herauszieht. Durch festeres Ergreifen der Einsatz-Seitenwände 28 beim Herausziehen des Einsatzes 2 aus dem Gehäuse 1 können auch etwaige Widerstände infolge von Wattekugeln 17 überwunden werden, die zwischen Gehäusewandung und Einsatz 2 gelangt sind und die Bewegung des letzteren hemmen.

Die Kanten 22 bis 24, und insbesondere die oberen Längskanten 22 der Aussparungen 21 dienen gleichzeitig als Griffkanten, mit deren Hilfe der Benutzer die Vorrichtung ohne Gefahr des Abrutschens desselbens leicht anheben und an eine andere Stelle versetzen kann.

Nachdem der leere Einsatz 2 der in der beschriebenen Weise aus dem Gehäuse 1 entfernt worden ist, kann eine neue Nachfüllpackung in der bereits beschriebenen Weise einfach und leicht in das Gehäuse 1 eingesetzt werden.

Es ist selbstverständlich, daß die im Ausführungsbeispiel gezeigte Zuordnung der Öffnungen 15, 16, 20, 21, 30 und 31 vom Fachmann in geeigneter Weise abgeändert werden kann. Beispielsweise kann die Einsetzöffnung in der Rückwand des Gehäuses 1 ausgebildet sein. Es ist auch möglich, die Entnahmeöffnung für die Wattekugeln in einer der Gehäuseflanken auszubilden und dementsprechend die der zugeordneten Durchtrittsöffnung gegenüberliegende Bodenöffnung des Einsatzes in der entsprechenden Einsatz-Seitenwand mit Ausrichtung auf die zugeordnete Flankenöffnung anzubringen. Diese Flankenöffnung dient dann zusammen mit der in der Einsatz-Seitenwand ausgebildeten Bodenöffnung als Durchgreiföffnungen zur Druckausübung auf das Bodenelement. Die Entnahmeöffnung und die Durchtrittsöffnung müssen nicht notwendigerweise auf die Bodenöffnungen ausgerichtet sein.

## Ansprüche

1. Vorrichtung zum Spenden von Wattekugeln mit einem Einsatz zur Aufnahme der Wattekugeln und einem Gehäuse zur Aufnahme des Einsatzes, wobei das Gehäuse eine Gehäusewandung, einen Gehäuseboden, eine Entnahmeöffnung für die Wattekugeln sowie eine Einsetzöffnung aufweist, durch die der Einsatz in das Gehäuse einsetzbar ist, und der Einsatz ein Einsatzgehäuse mit einer Durchtrittsöffnung für die Wattekugeln umfaßt, **dadurch gekennzeichnet,** daß in einer Vorderwand der Gehäusewandung (4) die Einsetzöffnung (20) sowie in wenigstens einer der sich an die Vorderwand anschließenden Gehäuseflanken (5) eine Flankenöffnung (21) ausgebildet ist.

2. Vorrichtung nach Anspruch 1, **dadurch gekennzeichnet,** daß die Einsetzöffnung (20) sich über die Gesamtbreite der Vorderwand erstreckt.

3. Vorrichtung nach Anspruch 1 oder 2, **dadurch gekennzeichnet,** daß die Einsetzöffnung (20) sich über die Gesamthöhe der Vorderwand erstreckt.

4. Vorrichtung nach wenigstens einem der Ansprüche 1 bis 3, **dadurch gekennzeichnet,** daß die Flankenöffnung (21) als eine in die Einsetzöffnung (20) mündende Aussparung (21) ausgebildet ist.

5. Vorrichtung nach wenigstens einem der Ansprüche 1 bis 4, **dadurch gekennzeichnet,** daß in jeder Gehäuseflanke (5) eine Flankenöffnung (21) ausgebildet ist.

6. Vorrichtung nach Anspruch 5, **dadurch gekennzeichnet,** daß beide Flankenöffnungen (21) gegenüberliegend angeordnet sind.

7. Vorrichtung nach wenigstens einem der Ansprüche 1 bis 6, dadurch gekennzeichnet, daß das Gehäuse (1) mit einem Zusatzgewicht (12) versehen ist.

8. Vorrichtung nach Anspruch 7, **dadurch gekennzeichnet,** daß das Zusatzgewicht (12) an einer der Einsetzöffnung (20) gegenüberliegenden Gehäuserückwand (7) angebracht ist.

9. Vorrichtung nach Anspruch 8, **dadurch gekennzeichnet,** daß die Gehäuserückwand (7) mit einer äußeren und einer inneren Rückwand (9, 10) doppelwandig ausgebildet und das Zusatzgewicht (12) im Zwischenraum (11) zwischen beiden Rückwänden (9, 10) angeordnet ist.

10. Vorrichtung nach wenigstens einem der Ansprüche 1 bis 9, **dadurch gekennzeichnet,** daß im Einsatz (2) ein in Richtung zur Durchtrittsöffnung (28) verschiebbares Bodenelement (32) angeordnet ist.

11. Vorrichtung nach Anspruch 10, **dadurch gekennzeichnet,** daß im Einsatzboden (26) und im Gehäuseboden (6) je eine Bodenöffnung (15, 31) als Durchgreiföffnungen zur Druckausübung auf das Bodenelement (32) ausgebildet sind.

12. Vorrichtung nach Anspruch 11, **dadurch gekennzeichnet,** daß die Bodenöffnungen (15, 31) bei in das Gehäuse (1) eingesetztem Einsatz (2) aufeinander ausgerichtet sind.

**FIG. 1**

FIG.2

FIG.3

FIG. 4

FIG.5

| | Europäisches Patentamt | EUROPÄISCHER RECHERCHENBERICHT | Nummer der Anmeldung |
| --- | --- | --- | --- |

EP  88 10 4006

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| --- | --- | --- | --- |
| A | US-A-4 365 709 (LESTER)<br>* Figuren 1,2; Anspruch 1 *<br>--- | 1 | A 61 F    15/00<br>A 45 D    44/00 |
| A | EP-A-0 093 315 (ROESCHEISEN GMBH & CO.)<br>* Anspruch 1; Figuren 1,12 *<br>--- | 1 | |
| A | US-A-2 699 780 (RUDNICK et al.)<br>* Figuren 1,3; Anspruch 1 *<br>--- | 1 | |
| D,A | US-A-2 136 352 (L'HOMMEDIEN)<br>* Figuren 1,4 *<br>--- | 1 | |
| A | US-A-2 426 911 (WILLIAMSON)<br>* Figuren 1-4,14-16 *<br>----- | 1 | |

**RECHERCHIERTE SACHGEBIETE (Int. Cl.4)**

A 45 D    44/00
A 61 F    15/00
B 65 D

Der vorliegende Recherchenbericht wurde für alle Patentansprüche erstellt

| Recherchenort | Abschlußdatum der Recherche | Prüfer |
| --- | --- | --- |
| BERLIN | 25-10-1988 | KANAL P K |